# EUROPEAN PATENT APPLICATION

(11) **EP 4 840 608 A1**
(43) Date of publication of application: **18.11.2026**
(21) Application number: 26177238.8
(22) Date of filing: 07.05.2026
(51) Int. Cl.: B29B 13/02, A61F 13/15, B25J 9/16

(54) **METHODS AND APPARATUSES FOR REPLENISHING HOT MELT MATERIALS FOR A MANUFACTURING CONVERTING LINE**

(30) Priority: 12.05.2025 US 202563803981 P
(71) Applicant: The Procter & Gamble Company, Cincinnati, OH 45202 (US)
(72) Inventor: SCHNEIDER, Uwe, Cincinnati, 45202 (US); ELSHARAWY, Mohamed, 65824 Schwalbach am Taunus (DE); HOANG, Tin Trung, 65824 Schwalbach am Taunus (DE)
(74) Representative: P&G Patent Germany

(57) **Abstract**

Methods for replenishing hot melt materials for use in one or more manufacturing converting lines. The hot melt material can be used to affix different materials and/or components to one another towards making a final product. Some of the methods utilize a driverless vehicle and an end effector to retrieve, and deliver to and/or load a hot melt material into a hot melt tank.

## Description

### CROSS REFERENCE TO RELATED APPLICATION

This application claims the benefit, under 35 U.S.C. §119(e), of U.S. Provisional Application No. 63/803,981, filed on May 12, 2025, the entire disclosure of which is hereby incorporated by reference herein.

### FIELD

The present disclosure relates to methods for replenishing hot melt material for use in manufacturing processes that combine the hot melt material with other materials and/or components to form final products. The present disclosure exemplifies apparatuses and tooling that can be used in these methods, but the methods are not limited to the same.

### BACKGROUND

Disposable absorbent products typically comprise several different materials and components, which can be affixed to one another with hot melt materials. The complexity and speed of modern manufacturing converting lines can however make it challenging to keep up with sufficient quantities of hot melt materials. This is exacerbated when a single converting line utilizes multiple different hot melt materials and/or when multiple converting lines are included within an manufacturing environment and require hot melt materials be replenished often.

It is not uncommon for a manufacturing converting line to utilize two or more different hot melt materials. The hot melt materials can differ in composition and therefore it is important to deliver and load the correct hot melt material into the appropriate hot melt tank. The hot melt materials can also differ in form factor when in their solid state form. For example, the hot melt materials may be supplied as a block of material or in aggregate form, which itself can take on different sizes and shapes like chips and pellets. Specific equipment may be necessary to deliver and load the different hot melt materials, leading to space, maintenance, safety and other issues.

Accordingly, there is a need for apparatuses and methods to improve the efficiency of replenishing hot melt materials to one or more manufacturing converting lines.

### SUMMARY

Methods for replenishing hot melt materials for one or more manufacturing converting lines are provided. The disclosed apparatuses and methods can be particularly useful in processes for making disposable absorbent products. Many of these products include a plurality of different hot melt materials, at least some of which comprise tackifiers and are employed to secure materials and/or components together. It should be understood that the disclosed apparatuses and methods can also be used to make products other than disposable absorbent products.

A first exemplary method includes, but is not limited to, the following steps: (a) signaling, via a fill-level sensing device associated with a hot melt material tank, a driverless vehicle to deliver a hot melt material; (b) delivering, via the driverless vehicle, the hot melt material to the hot melt material tank; and (c) loading, via an end effector associated with the driverless vehicle, the hot melt material into the hot melt material tank.

A second exemplary method includes, but is not limited to, the following steps: (a) providing a storage device comprising a first bin comprising a first hot melt material comprising a first composition and/or solid state form, and a second bin comprising a second hot melt material comprising a second composition and/or solid state form that is different from the first composition and/or solid state form; (b) moving the storage device from a first location to a second location that is closer to the manufacturing converting line; (c) signaling, via a fill-level sensing device associated with a first hot melt material tank, a driverless vehicle to retrieve the first hot melt material from the storage device; (d) moving the driverless vehicle proximate to the storage device; (e) identifying the bin containing the first hot melt material; (f) securing, via a first end effector associated with the driverless vehicle, the first hot melt material or the bin containing the first hot melt material from the storage device; (g) delivering, via the driverless vehicle, the first hot melt material to the first hot melt material tank; (h) loading, via the first end effector, the first hot melt material into the first hot melt material tank; (i) signaling, via a fill-level sensing device associated with a second hot melt material tank, the driverless vehicle to retrieve the second hot melt material from the storage device; (j) moving the driverless vehicle proximate to the storage device; (k) identifying the bin containing the second hot melt material; (l) securing, via the first end effector or a second end effector associated with the driverless vehicle, the second hot melt material or the bin containing the second hot melt material from the storage device; (m) delivering, via the driverless vehicle, the second hot melt material to the second hot melt material tank; and (n) loading, via the first end effector or the second end effector, the second hot melt material into the second hot melt material tank.

A third exemplary method includes, but is not limited to, the following steps: (a) providing a plurality of different types of hot melt materials; (b) providing a hot melt material storage device comprising a plurality of bins; (c) loading individual bins of the plurality of bins with individual types of hot melt materials; and (d) thereafter transferring the hot melt material storage device from a first location to a second location that is closer to the manufacturing converting line; (e) wherein the plurality of different types of hot melt materials have different configurations in their solid state within the bins; and (f) wherein the bins have substantially the same configuration such that a single tool can deliver the holt melt materials having different configurations to and/or load them into tanks associated with the manufacturing converting line.

### BRIEF DESCRIPTION OF THE DRAWINGS

The above-mentioned and other features and advantages of the present disclosure, and the manner of attaining them, will become more apparent and the disclosure itself will be better understood by reference to the following description of example forms of the disclosure taken in conjunction with the accompanying drawings, wherein:
FIG. 1 is a perspective view of an exemplary hot melt tank for converting a hot melt material from solid state to a flowable state conducive for application within a manufacturing process;
FIG. 2 is a perspective view of an exemplary driverless vehicle useful in the methods provided herein;
FIGS. 3A-3D are perspective views of exemplary bins for holding and/or loading different hot melt materials;
FIG. 4 is a perspective view of a driverless vehicle carrying two different bins of hot melt materials;
FIG. 5 is a perspective view of an exemplary storage rack containing a plurality of hot melt material bins;
FIG. 6 is a perspective view of a driverless vehicle having retrieved two bins of hot melt material from a moveable storage rack;
FIG. 7 is a perspective view of a driverless vehicle parked in front of a hot melt tank prior to loading additional material into the tank;
FIG. 8 is a partial perspective view to show the use of a robot arm and end effector to open a lid of a hot melt tank to allow additional hot melt material to be loaded therein;
FIG. 9 is a partial perspective view to show the use of an exemplary chute to facilitate loading hot melt material into a hot melt tank; and
FIGS. 10-12B are flow charts specifying steps of exemplary methods provided by the present disclosure.

### DETAILED DESCRIPTION

This disclosure is written to describe the invention to a person having ordinary skill in the art, who will understand that this disclosure is not limited to the specific examples or embodiments described. The examples and embodiments are single instances of the invention which will make a much larger scope apparent to the person having ordinary skill in the art. Unless defined otherwise, all technical and scientific terms used herein have the same meaning as commonly understood by the person having ordinary skill in the art. It is also to be understood that the terminology used herein is for the purpose of describing examples and embodiments only, and is not intended to be limiting, since the scope of the present disclosure will be limited only by the appended claims.

All the features disclosed in this specification (including any accompanying claims, abstract, and drawings) may be replaced by alternative features serving the same, equivalent, or similar purpose, unless expressly stated otherwise. Thus, unless expressly stated otherwise, each feature disclosed is one example only of a generic series of equivalent or similar features. The examples and embodiments described herein are for illustrative purposes only and that various modifications or changes in light thereof will be suggested to the person having ordinary skill in the art and are to be included within the spirit and purview of this application. Many variations and modifications may be made to the embodiments of the disclosure without departing substantially from the spirit and principles of the disclosure. All such modifications and variations are intended to be included herein within the scope of this disclosure. For example, unless otherwise indicated, the present disclosure is not limited to particular materials, reagents, reaction materials, manufacturing processes, or the like, as such can vary. It is also to be understood that the terminology used herein is for purposes of describing particular embodiments only and is not intended to be limiting. It is also possible in the present disclosure that steps can be executed in different sequence where this is logically possible.

All numeric values are herein assumed to be modified by the term "about," whether or not explicitly indicated. The term "about" generally refers to a range of numbers that one of skill in the art would consider equivalent to the recited value (for example, having the same function or result). In many instances, the term "about" may include numbers that are rounded to the nearest significant figure.

In everyday usage, indefinite articles (like "a" or "an") precede countable nouns and noncountable nouns almost never take indefinite articles. It must be noted, therefore, that, as used in this specification and in the claims that follow, the singular forms "a," "an," and "the" include plural referents unless the context clearly dictates otherwise. Thus, for example, reference to "a support" includes a plurality of supports. Particularly when a single countable noun is listed as an element in a claim, this specification will generally use a phrase such as "a single." For example, "a single support."

Where a range of values is provided, it is understood that each intervening value, to the tenth of the unit of the lower limit (unless the context clearly dictates otherwise), between the upper and lower limit of that range, and any other stated or intervening value in that stated range, is encompassed within the disclosure. The upper and lower limits of these smaller ranges may independently be included in the smaller ranges and are also encompassed within the disclosure, subject to any specifically excluded limit in the stated range. Where the stated range includes one or both of the limits, ranges excluding either or both of those included limits are also included in the disclosure.

In this specification and in the claims that follow, reference will be made to a number of terms that shall be defined to have the following meanings unless a contrary intention is apparent.

"Absorbent product " refers to devices that absorb and contain liquid, and more specifically, refers to devices that are placed against or in proximity to the body of the wearer to absorb and to contain various exudates discharged from the body.

"Disposable" refers to products which are designed to be discarded after a limited use rather than being laundered or otherwise restored for reuse.

"Bin", as used herein, means a receptacle for holding, storing material, and/or loading hot melt material. It is not limited to a particular size, shape, or configuration, and should be interpreted as an item onto and/or into which hot material can be placed.

The present invention provides methods for replenishing hot melt materials for use in manufacturing processes. "Hot melt material", as used herein, includes polymer-based materials that are applied to a material or component in a molten state (when heated, for example, to a temperature of about 200 to about 350 degrees Fahrenheit) and then cooled to a solid state. Exemplary polymers include, but are not limited to, ethylene-vinyl acetate (EVA), polyolefins, polyamides, polyesters, styrene block copolymers, polyethylene, ethylene-methyl acrylate (EMA), and ethylene n-butyl acrylate (EnBA).

The hot melt materials may include additives, such as, resins, waxes, antioxidants, plasticizers, fillers, and biocides. Tackifying resins can be employed to influence the adhesive nature of the holt melt material. Exemplary resins include rosin, hydrogenated rosin, C9, hydrogenated hydrocarbon, terpene phenolics, rosin ester, and C5. Waxes may be incorporated to influence the set speed and open time (amount of time to make a bond). Exemplary waxes include natural waxes, microcrystalline waxes, and synthetic waxes. Antioxidants can help protect the hot melt material from degrading, particularly during their shelf life and while heated in a supply tank. Exemplary antioxidants include phenols, aromatic amines, phosphates, phosphites, and butylated hydroxytoluene. The use of plasticizers in hot melt materials can provide good flexibility and toughness properties to the hot melt materials. Hydrocarbon oils that are low in aromatic content are one type of plasticizer suitable for use in the hot melt materials.

The hot melt materials used herein may contain bio-based materials and be biodegradable according to ASTM D6400 standards. It is preferred that the hot melt materials employed herein are substantially free or completely free of a solvent. Accordingly, hot melt materials can be an environmentally-friendly alternative to solvent-based adhesives. The bonding time and effectiveness of hot melt materials, particularly in dynamic manufacturing applications, can also make them preferred over solvent-based adhesives which can take longer to cure.

The hot melt materials can exhibit many different forms, including, but not limited to, pellets, chips, cakes, bags, bricks, drums, slats, and pillows. In some forms, the hot melt materials include packaging or casing material (for example, a bag) that does not have to be removed prior to loading the hot melt materials into a hot melt tank.

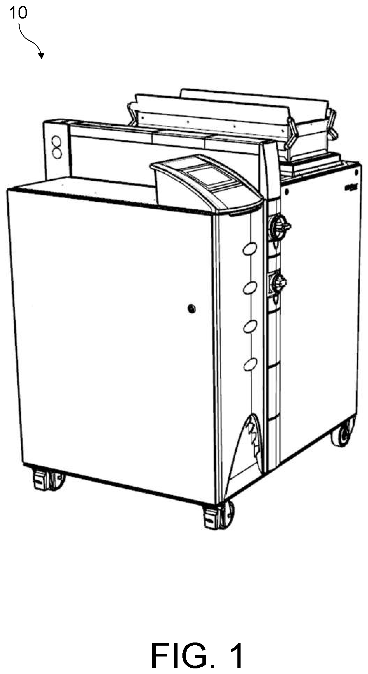

For continuous manufacturing processes, dedicated hot melt tanks are provided for each of the hot melt material compositions. The hot melt material is loaded in a solid state and is then melted to a desired viscosity before it is pumped or otherwise delivered via conduits to an application nozzle. An exemplary hot melt tank 10 is shown in FIG.1.

It is not unusual for a manufacturing converting line to employe two, three, four, five, six, or even more individual hot melt tanks for supplying a plurality of hot melt material applications to the various materials and components included and affixed to one another to make a final product.

Exemplary apparatuses and components thereof will now be described in connection with steps and job functions related to the inventive methods provided herein. It should be understood that while these exemplary apparatuses and components are useful for carrying out at least some of the inventive steps, the methods can be carried out by other apparatuses and components than those specifically shown and described. The apparatuses and methods herein can employ a driverless vehicle that is capable of securing hot melt materials from a storage location and delivering the same to one or more hot melt tanks. The vehicles typically include a controllable tool (for example, an end effector) that is capable of loading the delivered hot melt material into the appropriate holt melt tank.

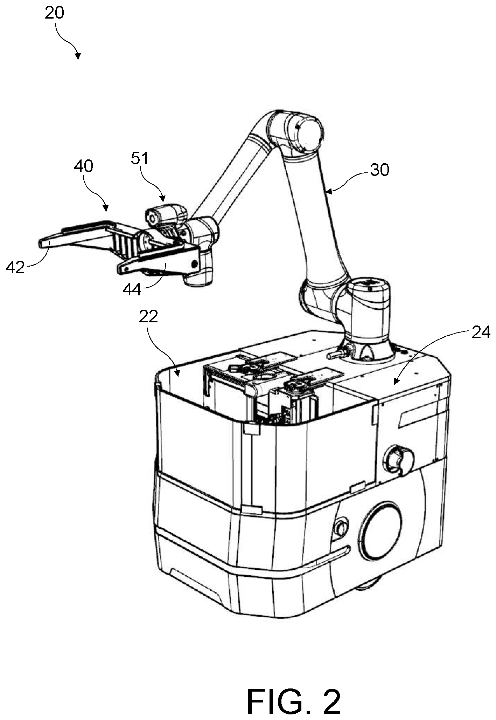

Suitable driverless vehicles include autonomous mobile robots, automated guided vehicles, gantry systems, car and rail/track systems, and the like. One exemplary driverless vehicle 20 is shown in FIG. 2, in the form of an autonomous mobile robot. Vehicle 20 includes a compartment 22 that can hold hot melt materials (in loose form or within a container), and/or tools, swappable end effectors, or other equipment to help load hot melt materials or perform maintenance on the vehicles or hot melt tanks, for example. Hot melt materials can also be transported on decking 24 of the vehicle; again, in their supplied form or within a container such as a bin.

Driverless vehicle 20 includes a robot arm 30 (for example, a six-axis robot arm) comprising an end effector 40, and optional camera 51. The exemplary end effector 40 includes spaced apart arms 42 and 44 for securing hot melt materials directly and/or bins containing the hot melt materials. At least one of arms 42 and 44 is moveable relative to the other for securing and thereafter releasing hot melt materials or containers holding the same. End effectors other than that shown in the figures herein can also be used in the methods of the present invention.

The fill-level of a hot melt tank is typically monitored, and an alert or other indication will be communicated that additional hot melt material is needed to supply a running manufacturing converting line. The alert/indication can be reacted to by a human operator who in turn communicates a request that more hot melt material be delivered to a hot melt tank. In one form, an alert/indication can automatically lead to a signal that is communicated wirelessly to a driverless vehicle, such as that shown in FIG. 2, requesting the driverless vehicle to retrieve and deliver additional hot melt material. The hot melt tank fill-level can be detected by a sensor (for example, a radar sensor, a laser sensor, or a weight sensor) associated with the hot melt tank.

The hot melt tank fill-level may alternatively be monitored via a separate control system that may or may not be in communication with the hot melt tank. Thus, the hot melt tank fill-level can be determined through calculations related to input and throughput versus be actually measured or detected. In one form, a tank fill-level signal is sent to a control system and vehicle fleet manager, which chooses and triggers action of a suitable vehicle to retrieve, deliver and/or load hot melt material into a specific hot melt tank. A control system can be employed to monitor fill levels of multiple hot melt tanks associated with one and/or several manufacturing converting lines. In this scenario, the hot melt tank with the least remaining run time is prioritized and the control system signals a vehicle to perform the hot melt material retrieval and loading steps.

A signal from the hot melt tank and/or control system will contain information regarding which hot melt material is needed. If not already in a storage area, the driverless vehicle will move to an area storing different hot melt materials. Individual hot melt materials will be stored in a known position and/or may contain a unique identifier to aid the driverless vehicle securing the correct hot melt material that is being requested. Unique identifiers can include, for example, barcodes, QR codes, tags as part of RFID systems, alphanumeric characters, color codes, etc. In one form, the unique identifiers can be read by a camera (for example, camera 51 of robot arm 30) or a separate reader associated with the driverless vehicle. Once the correct hot melt material has been identified by the driverless vehicle, an end effector (for example, end effector 40) secures the hot melt material itself or a container containing the hot melt material.

The hot melt materials may be taken from their shipper container used by the hot melt material manufacturer or distributor, and subsequently put into smaller containers (for example, bins) for transport from a storage area to a hot melt tank or staging area proximate to a manufacturing converting line utilizing the hot melt material. These bins can include a unique identifier associated with a specific hot melt material composition. The unique identifier may be digital or in another form that is easily changeable so that the bins can be reused with different hot melt materials.

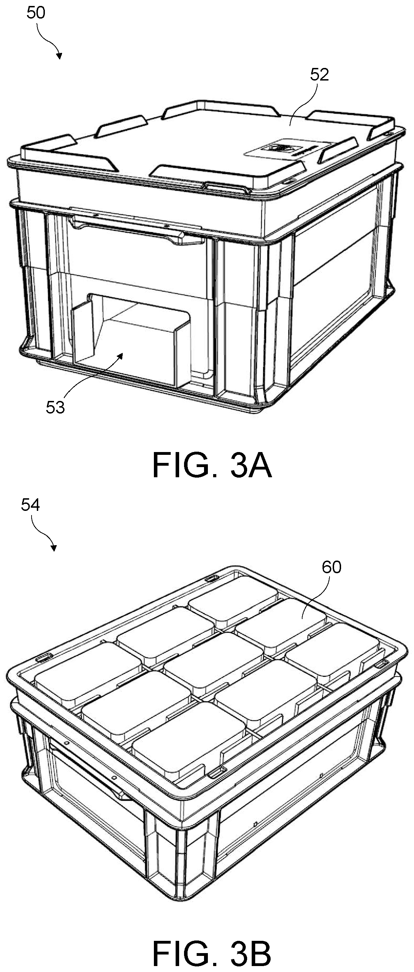

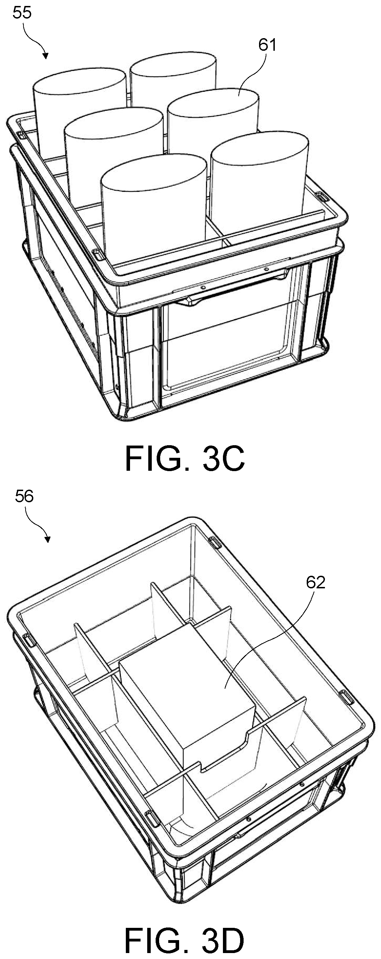

Exemplary bins are illustrated in FIGS. 3A-3D. Bin 50, in FIG. 3A, is shown with an optional, removable lid 52 and an open or openable portion 53. The open or openable portion can allow hot melt materials to exit the bins during loading without having to completely invert the bin; however, complete or near complete inversion of the bins during loading a hot melt tank is also contemplated herein. Lids can be employed with the bins to help prevent contamination from entering the bins prior to the hot melt materials being loaded into hot melt tanks. Lids can also facilitate stacking of the bins. Bins 54, 55, and 56, of FIGS. 3B, 3C, and 3D, respectively, are shown with different sized and/or configured hot melt materials 60, 61 and 62. Exemplary bins can have internal pockets that are all the same, as shown with bins 54 and 55 in FIGS. 3B and 3C, or have different sized and/or configured pockets like that shown in bin 56 in FIG. 3D.

The individual bins containing different hot melt materials can be substantially the same configuration wherein a single tool or end effector is capable of securing, transporting, and/or loading hot melt materials having a different solid state form factor. For example, multiple bins can comprise generally the same shape, such as the rectangular bins shown in FIGS. 3A-3D. Multiple bins can also comprise a portion having a similar dimension/configuration, but have other portions with dissimilar dimensions or configurations, wherein a single tool can secure the different bins about the portion having the similar dimension/configuration. By way of example only, bin 50 shares a common width dimension with bin 54, while their respective height dimensions are different.

In one embodiment, a single end effector is capable of handling all of the different hot melt materials used in a manufacturing process. In another embodiment, two or more different end effectors are utilized in association with a driverless vehicle to secure, transport, and/or load different hot melt materials. The two or more different end effectors may be configured for securing differently configured bins, or alternatively, one end effector may be configured for securing a bin holding a hot melt material while the second end effector is configured for securing the holt melt material directly in the absence of, or out of, a bin.

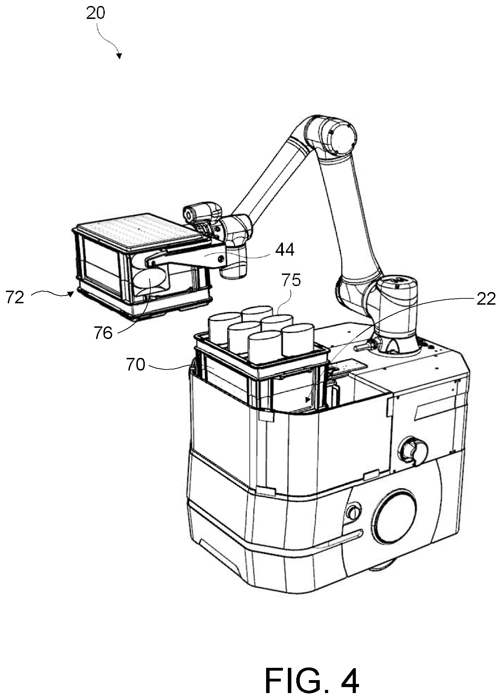

FIG. 4 shows driverless vehicle 20 having secured two bins 70 and 72 containing hot melt material. Bin 70 contains a first hot melt material 75, and is located within compartment 22 of vehicle 20. Bin 72 contains a second hot melt material 76, and is being held by end effector arms 42 (shown in FIG. 2) and 44. First hot melt material 75 can be different from second hot melt material 76 in composition, set speed, open time, melting point, viscosity at a given temperature, shape, size, volume, mass, or other. In accordance with one aspect, the two hot melt materials are different from one another in at least two of composition, set speed, open time, melting point, viscosity at a given temperature, shape, size, volume and mass. Alternatively, bins 70 and 72 could contain the same hot melt material, with them both being delivered to a single manufacturing converting line or two different manufacturing converting lines located within a common manufacturing environment.

In one aspect, a first hot melt material comprises a first mass and a second hot melt material comprises a second mass, wherein a ratio of the first mass to the second mass is at least 2:1, 3:1, 5:1, 10:1, 20:1, or 30:1. In another aspect, a first hot melt material comprises a first volume and a second hot melt material comprises a second volume, wherein a ratio of the first volume to the second volume is at least 2:1, 3:1, 5:1, or 10:1. When evaluating mass and volume of different hot melt materials, it is to be understood that it relates to distinct hot melt material pieces, and not an aggregate of distinct pieces that may be contained in a bag or casing for example. And the above-described ratios may be calculated on a single piece comparison and also on an average basis that contemplates multiple measured pieces.

Storage areas for receiving and holding raw materials, such as hot melt materials, used in a manufacturing environment may be a significant distance away from the converting lines processing the raw materials. In this scenario, it can be useful to move materials from an initial storage location to a second location that is closer to the manufacturing converting line. Furthermore, the frequency of delivering and loading hot melt materials may necessitate utilizing such an intermediate material staging area rather than moving back and forth longer distances to secure hot melt materials. Thus, a movable storage container that includes a plurality of bins containing different hot melt materials may be employed in the methods provided herein. A moveable storage container can be transported from a prep area where hot melt materials are loaded into bins and the bins loaded into the storage container, to a location closer to the converting line(s). Hot melt materials can be loaded into the bins, and the bins loaded into the moveable storage container, by humans or robots, for example. When robots are used for loading the bins into the moveable storage container, a robot associated with the driverless vehicle may be used, or a separate robot may be used.

Pre-dosing hot melt material into bins by humans or robots unassociated with a driverless vehicle, followed by transporting the bins to a staging area, can provide a number of benefits. Hot melt material shipper containers can be rather large and storing them next to a hot melt tank of a manufacturing converting line can be spaced constrained. Additionally, discrete hot melt material forms may be sticking to teach other, especially when compressed at the bottom of a shipper container, making it difficult for a robot arm/end effector configured for loading the hot melt material into a hot melt tank to secure an agglomerated amount of hot melt material. Furthermore, a reach of a robot arm associated with a driverless vehicle may not be sufficient to secure all of the hot melt material from a large shipper container. Pre-dosing specific volumes of hot melt material into a bin may also speed up the process of loading a hot melt tank.

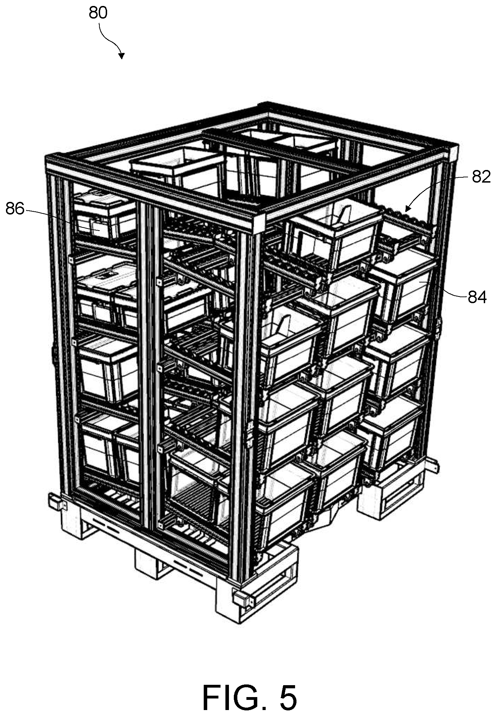

An exemplary moveable storage container in the form of a rack 80 is shown in FIG. 5. Rack 80 can be moved from its prep area to a second location proximate the converting line by a vehicle, which can be the same or a different driverless vehicle than that used to deliver the bins from rack 80 to a hot melt tank. Storage rack 80 can also be moved by a vehicle operated by a driver; for example, a human operated fork lift. In one form, an automated guided vehicle is employed to move rack 80 from its prep area to the second location, while automated mobile robot 20 is employed to secure individual bins from rack 80 and deliver the contents thereof to a hot melt tank.

Storage rack 80 contains individual storage areas 82 for containing individual bins 84 and 86. As shown in FIG. 5, bin 84, while configured similarly to bin 86, is a different size than bin 86. Bins 84 and 86 may contain the same hot melt material or different hot melt materials. Different hot melt materials can, again, differ in composition (with corresponding physical properties manifested by the compositions), shape, size, volume, and/or mass. The similar configuration of bins 84 and 86 allow a single end effector to manipulate the bins. It should be understood that while exemplary storage rack 80 shows bins having a similar overall configuration, bins of different configurations may be loaded into the rack and secured by one or more end effectors. It should also be understood that moveable storage containers other than a rack can be utilized.

In one form, an end effector secures a bin containing hot melt material from storage rack 80 and delivers the same to hot melt tank. In an alternative form, an end effector can retrieve hot melt material from a bin while it is disposed within storage rack 80, and transport the hot melt material from storage rack 80 while leaving the bin behind.

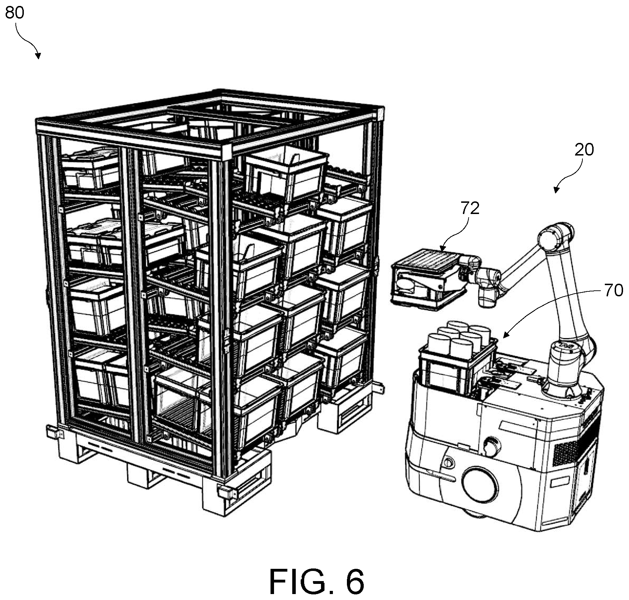

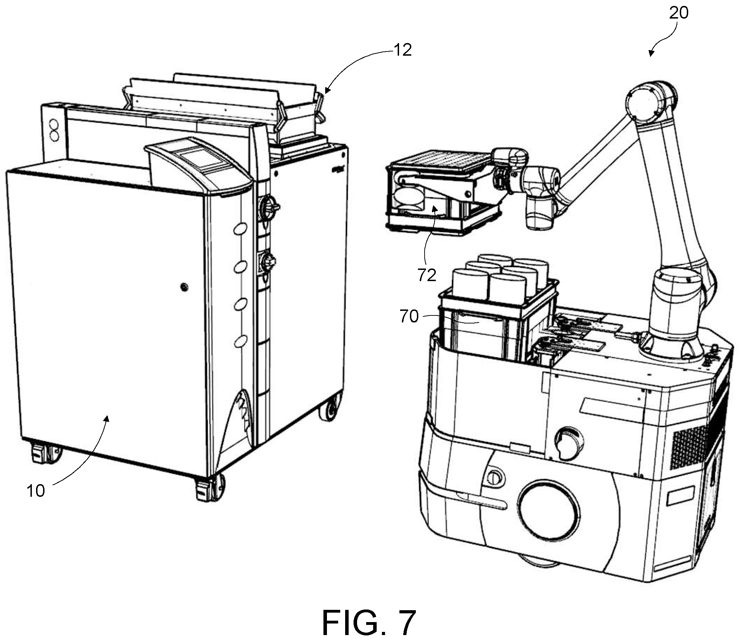

FIG. 6 shows exemplary driverless vehicle 20 with two bins 70 and 72 containing hot melt materials secured from storage rack 80. Driverless vehicle 20 then delivers the hot melt materials to a hot melt tank 10, as shown in FIG. 7. Hot melt tank 10 includes a lid 12 that is opened as vehicle 20 approaches or is parked in front of the tank. Lid 12 can automatically open when a sensor (on the floor, vehicle, or hot melt tank) is triggered by the approaching vehicle 20. Notice that lid 12, when opened, also creates a funnel to aid in the loading of the hot melt material. Funnels or load assist members can also be used that are not part of the hot melt tank lid/cover.

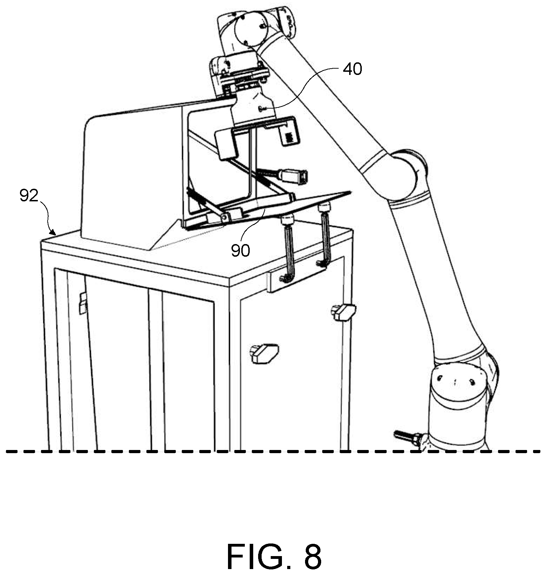

While a hot melt tank lid can be automatically opened by built-in mechanisms, a hot melt tank lid can be manually opened via a robot associated with the driverless vehicle. FIG. 8 shows a lid 90 (or access cover) of hot melt tank 92 that is opened by end effector 40. In a scenario where a robot manually opens a hot melt tank lid prior to loading hot melt material, a tool exchange may take place wherein a first end effector opens the hot melt tank lid and a second end effector loads (directly or by dumping a bin) the hot melt material. Lids may close via counterweights after the hot melt material is loaded.

Lids, access port covers, or the like may be designed to be in a normally biased closed position to help prevent contamination from entering a hot melt tank and to maintain control of the elevated internal temperature. The lids or access port covers pivot out of the way when hot melt materials are placed against them, such that it is the mass of the hot melt material that opens the lid or access port cover. Once the hot melt material passes through, the lid or access port then recloses based on its biasing mechanism. Thus, the lid or access port with this configuration acts like a one-way valve or check valve.

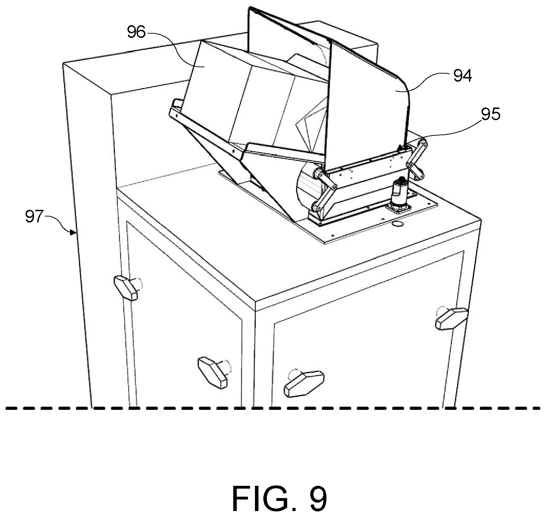

FIG. 9 shows a load assist member in the form of an openable chute 94 that can be temporarily or permanently positioned within a hot melt tank lid or access port 95 and that facilitates loading of hot melt material 96 into holt melt tank 97.

In a separate embodiment, a driverless vehicle does not contain a robot arm and it is simply used to deliver loaded hot melt material from a storage area to a hot melt tank. The hot melt material is loaded onto the driverless vehicle and/or unloaded from the driverless vehicle by other means; for example, human operators, robots, or other equipment.

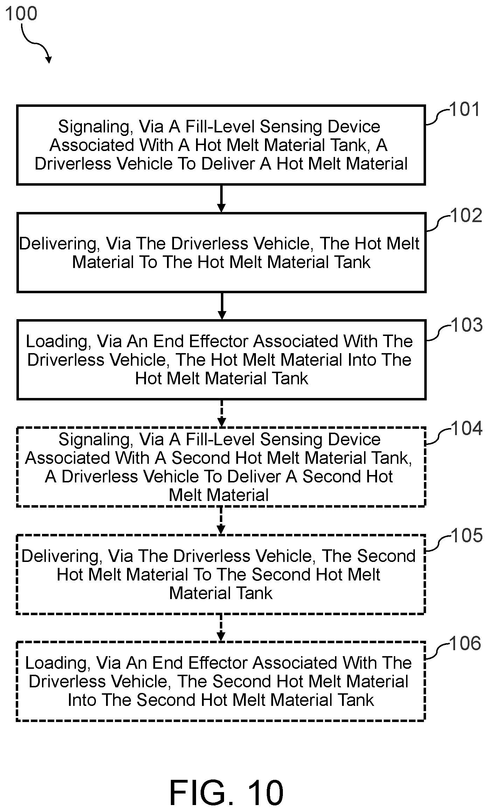

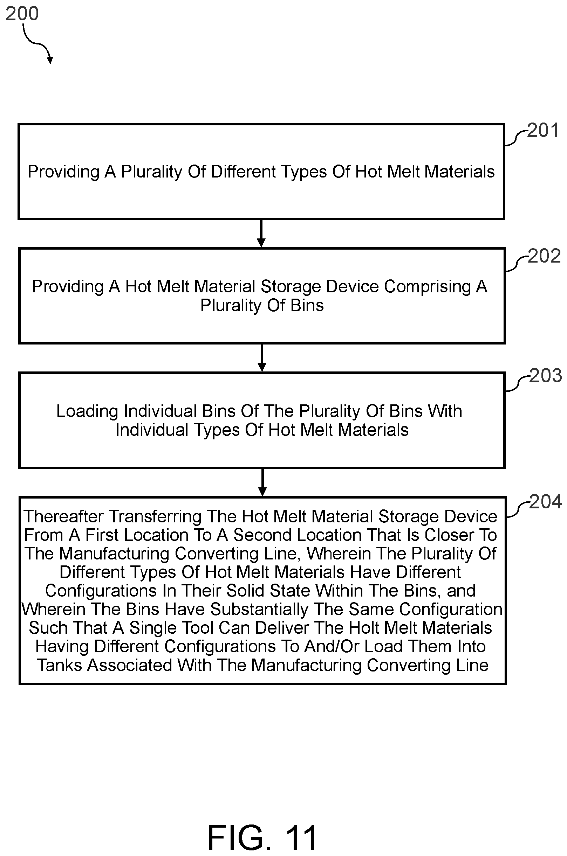

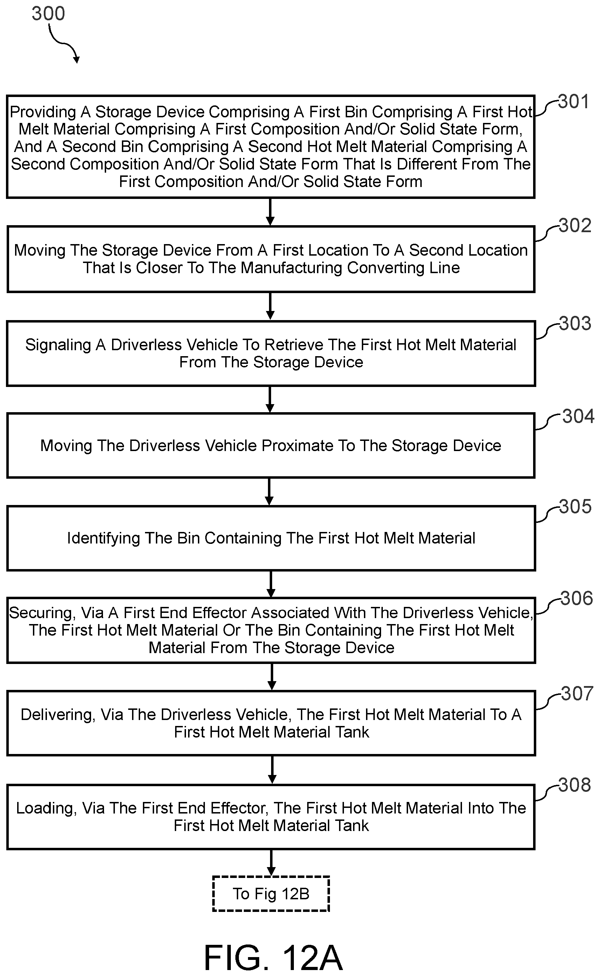

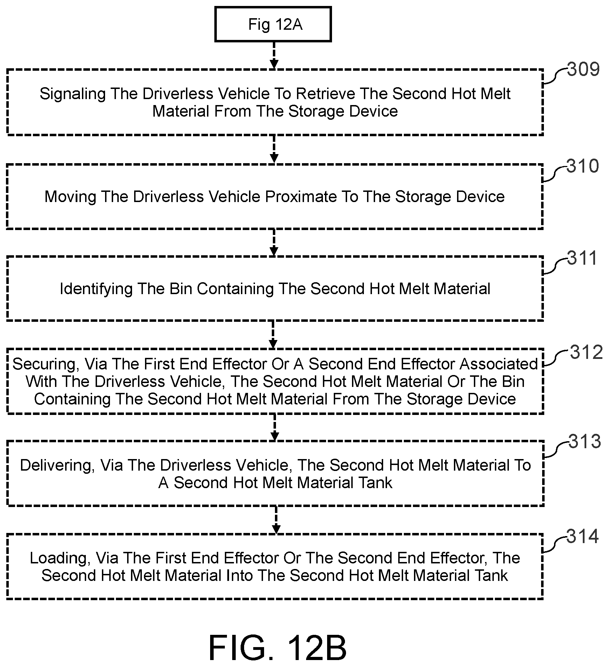

FIGS. 10, 11 and 12 illustrate three exemplary methods 100, 200, and 300, respectively, according to the present disclosure. Steps of methods 100, 200, and 300 are labeled with consecutive numbers. The steps included in broken line text boxes are optional steps in the methods. It should be understood that the method steps included in FIGS. 10, 11, and 12 can be at least partially completed with the equipment shown in FIGS. 1-8, but can alternatively be completed with variations to the equipment shown in FIGS. 1-8 or with different equipment altogether. It should also be understood that additional steps can be included in exemplary methods 100, 200, and 300, and that individual steps may be varied to what is listed, or even eliminated altogether.

Robot arms and end effectors are shown and described as being associated with a vehicle, such as an autonomous mobile robot or automated guided vehicle. However, the robot arms and end effectors can be associated with other systems that enable their movement; for example, a gantry system that is positioned above one or more manufacturing converters, or a linear rail robot moveable relative to a converting line. Robot arms and end effectors can also be substantially stationary and positioned in close proximity to a manufacturing converter, with hot melt material brought to their location by a driverless vehicle for loading into a hot melt tank.

Apparatuses/vehicles suitable for use in the disclosed methods can comprise a variety of components and capabilities, depending on the desired steps or aspects of a particular strand material managing method. Vehicles typically comprise one or more batteries for powering the vehicle and robot arm(s). The batteries can be exchanged automatically by a mechanism associated with the vehicle (for example a robot arm and end effector), or a mechanism associated with a battery storage and replacement station. Batteries can also be exchanged by humans. In one form, the batteries are inductively chargeable with one or more charging mats positioned on the floor in the manufacturing environment. Battery charging mats can be included at various locations, including, for example, proximate a spool storage area or proximate an unwinder so that batteries can be charged simultaneously with other jobs being completed with the vehicles and accompanying robot end effectors. Battery charging mats or stations can also be positioned in a location requiring the vehicle to navigate different paths than those taken to retrieve and/or load new spools of strand material. In one form, the vehicles comprise two distinct types of batteries, one of which can be charged via contact charging and the other can be charged inductively.

The vehicles and/or accompanying components can include sensors adapted for a variety of benefits. Safety sensors (for example, ultrasonic sensors) can be employed to prevent a moving vehicle from contacting or negatively interacting with humans or equipment. The safety sensors can be selectively activated and deactivated. For example, safety sensors can be deactivated when the vehicle is in, or close to, a position to retrieve hot melt material from a storage location or close to a position that is in front of a hot melt tank. The safety sensors can be reactivated once the vehicle moves away from the equipment with which it previously interacted.

A position of the vehicles can be determined within a manufacturing environment for both safety (as noted above) and for efficiently carrying out various jobs. The position can be determined by, for example, GPS or readable elements, such as barcodes, which are associated with the vehicle and/or its accompanying components.

The vehicles and/or accompanying components can include one or more cameras. Cameras on a vehicle or robot end effector can be employed to aid in engaging a hot melt material or bin containing the same and/or loading the hot melt material into a hot melt tank.

The vehicles and/or their accompanying components may be able to connect to equipment with which they interact that enables the transfer of compressed air, vacuum, power, lubrication, or data, for example. Such connection can be a simple hose connection or even a docking station that allows multiple different transfers therebetween.

The manufacturing environment may include a "garage" where the vehicles can park for various maintenance purposes. For example, the vehicles can be lifted so that their wheels can be cleaned. Many of the materials used in the manufacture of disposable absorbent products can lint or dust. The vehicle wheels coefficient of friction can change as they accumulate lint/dust or other debris, which can in turn cause the wheels to slip. Debris can also accumulate on and around sensors and cameras, causing them to operate less than optimally. Thus, pressurized air, for example, can be used to clean sensors and cameras while the vehicle is parked in the garage. A vehicle may include an on-board pressure vessel which can also be filled when the vehicle is parked in the garage. Pressurized air from a charged pressure vessel can then be used for actuating pneumatic actuators, or for cleaning the vehicle and its components, for example. The vehicle batteries can also be charged while the vehicle is parked in the garage. Again, this can be done via contact charging and/or inductive charging (for example, wireless charging from below, the side, or above the vehicle). Additional maintenance items can also be conducted while the vehicle is parked in the garage. By way of example only, parts can be replaced or lubricated.

### Examples/Combinations

A. A method for replenishing hot melt materials for multiple manufacturing converting lines, the method comprising the steps of:
   (a) providing a driverless vehicle comprising an end effector;
   (b) signaling the driverless vehicle to deliver a hot melt material to a first manufacturing converting line;
   (c) delivering, via the driverless vehicle, the hot melt material to a hot melt material tank supplying the first manufacturing converting line;
   (d) loading, via the end effector, the hot melt material into the hot melt material tank supplying the first manufacturing converting line;
   (e) signaling the driverless vehicle to deliver the hot melt material to a second manufacturing converting line;
   (f) delivering, via the driverless vehicle, the hot melt material to a hot melt material tank supplying the second manufacturing converting line; and
   (g) loading, via the end effector, the hot melt material into the hot melt material tank supplying the second manufacturing converting line.
B. The method according to Paragraph A, wherein the hot melt material is contained within a storage device located on the driverless vehicle.
C. The method according to Paragraph A, wherein the hot melt material is retrieved from a storage location before step (c) and step (f).
D. A method for replenishing hot melt materials for a manufacturing converting line, the method comprising the steps of:
   (a) providing a storage device comprising a plurality of bins, individual bins of which contain a hot melt material therein;
   (b) moving the storage device to a location proximate the manufacturing converting line;
   (c) signaling a request to a driverless vehicle to retrieve a holt melt material from the storage device;
   (d) identifying a bin containing the requested hot melt material;
   (e) securing the bin containing the requested hot melt material;
   (f) delivering, via the driverless vehicle, the requested hot melt material to a hot melt material tank; and
   (g) loading the hot melt material into the hot melt material tank.
E. A method for replenishing hot melt materials for a manufacturing converting line, the method comprising the steps of:
   (a) providing a driverless vehicle comprising an end effector;
   (b) signaling the driverless vehicle to deliver a first hot melt material to a first hot melt material tank;
   (c) delivering, via the driverless vehicle, the first hot melt material to the first hot melt material tank;
   (d) loading, via the end effector, the first hot melt material into the first hot melt material tank;
   (e) signaling the driverless vehicle to deliver a second hot melt material to a second hot melt material tank;
   (f) delivering, via the driverless vehicle, the second hot melt material to the second hot melt material tank; and
   (g) loading, via the end effector, the second hot melt material into the second hot melt material tank;
   (h) wherein the first hot melt material is different from the second hot melt material.
F. A method for replenishing hot melt materials for a manufacturing converting line, the method comprising the steps of:
   (a) determining or detecting an amount of a first hot melt material that is remaining in a first tank as part of the manufacturing converting line;
   (b) sending a signal to a driverless vehicle to retrieve an additional amount of the first hot melt material;
   (c) loading, via an end effector associated with the driverless vehicle, the additional amount of the first holt melt material into the first tank;
   (d) determining or detecting an amount of a second hot melt material that is remaining a second tank as part of the manufacturing converting line;
   (e) sending a signal to the driverless vehicle to retrieve an additional amount of the second hot melt material; and
   (f) loading, via an end effector associate with the driverless vehicle, the additional amount of the second hot melt material into the second tank;
   (g) wherein the first hot melt material is different from the second hot melt material in at least two of composition, shape, size, volume, and mass.
G. A method for replenishing hot melt materials for a manufacturing converting line, the method comprising the steps of:
   (a) delivering a first hot melt material, via a driverless vehicle, to the manufacturing converting line; and
   (b) delivering a second hot melt material, via the same driverless vehicle, to the manufacturing converting line;
   (c) wherein the first hot melt material is different from the second hot melt material in at least two of composition, volume, shape, size, and mass.
H. A method for replenishing hot melt materials for a manufacturing converting line, the method comprising the steps of:
   (a) delivering a hot melt material to a staging area with a first driverless vehicle;
   (b) retrieving the hot melt material from the staging area with a second driverless vehicle that is different from the first driverless vehicle; and
   (c) delivering the hot melt material, via the second driverless vehicle, to the manufacturing converting line.
I. A method for replenishing hot melt materials for a manufacturing converting line, the method comprising the steps of:
   (a) providing a storage device comprising a plurality of storage areas;
   (b) loading a first hot melt material and a second hot melt material into individual storage areas of the plurality of storage areas; and
   (c) wherein the individual storage areas comprise a unique identifier associated with the respective first hot melt material and second hot melt material;
   (d) wherein the first hot melt material is different from the second hot melt material in at least two of composition, volume, shape, size, and mass;
   (e) retrieving, via a driverless vehicle, the first hot melt material from the storage device and delivering it, via the driverless vehicle, to the manufacturing converting line; and
   (f) retrieving, via a driverless vehicle, the second hot melt material from the storage device and delivering it, via the driverless vehicle, to the manufacturing converting line.
J. A method for replenishing hot melt materials for a manufacturing converting line, the method comprising the steps of:
   (a) determining or detecting an amount of a hot melt material remaining in a tank that is part of a continuous manufacturing converting line;
   (b) retrieving, via a driverless vehicle comprising a robot arm an end effector, additional hot melt material from a storage location;
   (c) delivering, via the driverless vehicle, the additional hot melt material in solid form to the tank;
   (d) opening an access port to the tank; and
   (e) loading, via the robot arm and end effector, the additional hot melt material in solid form into the tank through the access port.
K. A method for replenishing hot melt materials for a manufacturing converting line, the method comprising the steps of:
   (a) providing a driverless vehicle comprising a robot arm and end effector;
   (b) sending a signal to the driverless vehicle that a first hot melt material is needed in a first hot melt material tank;
   (c) delivering, via the driverless vehicle, the first hot melt material to the first hot melt material tank;
   (d) sending another signal to the driverless vehicle that a second hot melt material is needed in a second hot melt material tank; and
   (e) delivering, via the driverless vehicle, the second hot melt material to the second hot melt material tank;
   (f) wherein the first hot melt material is different from the second hot melt material in composition and at least one of size, shape, mass, and volume.

1. A method for replenishing hot melt materials for a manufacturing converting line, the method comprising the steps of:
   (a) signaling, via a fill-level sensing device associated with a hot melt material tank, a driverless vehicle to deliver a hot melt material;
   (b) delivering, via the driverless vehicle, the hot melt material to the hot melt material tank; and
   (c) loading, via an end effector associated with the driverless vehicle, the hot melt material into the hot melt material tank.
2. The method according to paragraph 1, wherein the driverless vehicle is an autonomous mobile robot.
3. The method according to paragraph 1 or 2, wherein the hot melt material is contained within a bin that is secured by the end effector.
4. The method according to any one of paragraphs 1-3, wherein the bin comprises an open or openable portion that is opened to complete step (c).
5. The method according to any one of paragraphs 1-4, wherein the driverless vehicle comprises a storage device comprising a plurality of bins containing the hot melt material.
6. The method according to any one of paragraphs 1-5, comprising a step (d), prior to step (b), of retrieving the hot melt material from a storage device comprising a plurality of bins, at least one of which contains the hot melt material.
7. The method according to paragraph 6, wherein the plurality of bins have substantially the same configuration but the hot melt material within individual bins of the plurality of bins have a different configuration from one another.
8. The method according to paragraph 6 or 7, wherein the storage device is loaded by a robot or a human.
9. The method according to any one of paragraphs 6-8, comprising a step (e), prior to step (d), of transporting the storage device from a first location to a second location that is closer to the manufacturing converting line.
10. The method according to paragraph 9, wherein step (e) is completed with a second driverless vehicle.
11. The method according to paragraph 10, wherein the second driverless vehicle is different from the driverless vehicle used in steps (a) to (c).
12. The method according to paragraph 9 or 10, wherein the second driverless vehicle is an automated guided vehicle.
13. The method according to any one of paragraphs 1-5, comprising steps of:
   (d) signaling, via a fill-level sensing device associated with a second hot melt material tank, a driverless vehicle to deliver a second hot melt material;
   (e) delivering, via the driverless vehicle, the second hot melt material to the second hot melt material tank; and
   (f) loading, via an end effector associated with the driverless vehicle, the second hot melt material into the second hot melt material tank.
14. The method according to paragraph 13, wherein the first hot melt material tank supplies a first manufacturing converting line, wherein the second hot melt material tank supplies a second manufacturing converting line, and wherein the first hot melt material is the same as the second hot melt material.
15. The method according to paragraph 13, wherein both of the first hot melt material tank and the second hot melt material tank supply a single manufacturing converting line, and wherein the first hot melt material is different from the second hot melt material.
16. The method according to paragraph 13 or 15, wherein the first hot melt material is different from the second hot melt material in composition and solid-state product form.
17. The method according to any one of paragraphs 13, 15, and 16, wherein the first hot melt material comprises a first composition and a first mass, wherein the second hot melt material comprises a second composition and a second mass, and wherein a ratio of the first mass to the second mass is at least 5:1.
18. The method according to any one of paragraphs 13 and 15-17 wherein the first hot melt material comprises a first composition and a first volume, wherein the second hot melt material comprises a second composition and a second volume, and wherein a ratio of the first volume to the second volume is at least 5:1.
19. The method according to any one of paragraphs 13 and 15-18, wherein each of the first hot melt material and the second hot melt material are stored in a bin associated with a storage device comprising a plurality of bins, wherein the bin containing the first hot melt material has a first unique identifier and wherein the bin containing the second hot melt material has a second unique identifier so that the driverless vehicle can accurately complete steps (b), (c), (e), and (f).
20. A method for replenishing hot melt materials for a manufacturing converting line, the method comprising the steps of:
   (a) providing a storage device comprising a first bin comprising a first hot melt material comprising a first composition and/or solid state form, and a second bin comprising a second hot melt material comprising a second composition and/or solid state form that is different from the first composition and/or solid state form;
   (b) moving the storage device from a first location to a second location that is closer to the manufacturing converting line;
   (c) signaling a driverless vehicle to retrieve the first hot melt material from the storage device;
   (d) moving the driverless vehicle proximate to the storage device;
   (e) identifying the bin containing the first hot melt material;
   (f) securing, via a first end effector associated with the driverless vehicle, the first hot melt material or the bin containing the first hot melt material from the storage device;
   (g) delivering, via the driverless vehicle, the first hot melt material to a first hot melt material tank;
   (h) loading, via the first end effector, the first hot melt material into the first hot melt material tank;
   (i) signaling the driverless vehicle to retrieve the second hot melt material from the storage device;
   (j) moving the driverless vehicle proximate to the storage device;
   (k) identifying the bin containing the second hot melt material;
   (l) securing, via the first end effector or a second end effector associated with the driverless vehicle, the second hot melt material or the bin containing the second hot melt material from the storage device;
   (m)delivering, via the driverless vehicle, the second hot melt material to a second hot melt material tank; and
   (n) loading, via the first end effector or the second end effector, the second hot melt material into the second hot melt material tank.
21. The method according to paragraph 20, wherein the first bin and the second bin are loaded by a robot or a human.
22. The method according to paragraph 20 or 21, wherein step (b) is completed with a second driverless vehicle.
23. The method according to paragraph 22, wherein the second driverless vehicle is different from the driverless vehicle used in steps (c) to (n).
24. The method according to paragraph 22 or 23, wherein the second driverless vehicle is an automated guided vehicle.
25. The method according paragraph 20 or 21, wherein the driverless vehicle employed in steps (c) to (n) is an autonomous mobile robot.
26. The method according to paragraph 25, wherein the autonomous mobile robot actuates a lid opening device associated with each of the first hot melt material tank and the second hot melt material tank.
27. The method according to any one of paragraphs 20-26, wherein the first end effector opens a lid of the first hot melt material tank prior to initiating step (h).
28. The method according to any one of paragraphs 20-27, wherein the first end effector and the second end effector are the same.
29. The method according to any one of paragraphs 20-28, wherein each of the first bin and the second bin comprises an openable portion that partially enables the completion of steps (h) and (n).
30. The method according to any one of paragraphs 20-29, further comprising a step (o) of moving the driverless vehicle to a battery charging station to charge one or more batteries providing power to the driverless vehicle and/or end effector associated with the driverless vehicle.
31. The method according to any one of paragraphs 20-29, further comprising a step (o) of moving the driverless vehicle to a battery exchange station to swap out one or more batteries providing power to the driverless vehicle and/or end effector associated with the driverless vehicle.
32. The method according to any one of paragraphs 20-31, wherein a load assistant device is used to complete at least one of step (h) and step (n).
33. The method according to paragraph 32, wherein the load assistant device comprises a funnel.
34. A method for replenishing hot melt materials for a manufacturing converting line, the method comprising the steps of:
   (a) providing a plurality of different types of hot melt materials;
   (b) providing a hot melt material storage device comprising a plurality of bins;
   (c) loading individual bins of the plurality of bins with individual types of hot melt materials; and
   (d) thereafter transferring the hot melt material storage device from a first location to a second location that is closer to the manufacturing converting line;
   (e) wherein the plurality of different types of hot melt materials have different configurations in their solid state within the bins; and
   (f) wherein the bins have substantially the same configuration such that a single tool can deliver the holt melt materials having different configurations to and/or load them into tanks associated with the manufacturing converting line.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A method for replenishing hot melt materials for a manufacturing converting line, the method comprising the steps of:
(a) signaling, via a fill-level sensing device associated with a hot melt material tank, a driverless vehicle to deliver a hot melt material;
(b) delivering, via the driverless vehicle, the hot melt material to the hot melt material tank; and
(c) loading, via an end effector associated with the driverless vehicle, the hot melt material into the hot melt material tank.

2. The method according to claim 1, wherein the driverless vehicle is an autonomous mobile robot.

3. The method according to claim 1 or 2, wherein the hot melt material is contained within a bin that is secured by the end effector.

4. The method according to any one of the preceding claims, wherein the bin comprises an open or openable portion that is opened to complete step (c).

5. The method according to any one of the preceding claims , wherein the driverless vehicle comprises a storage device comprising a plurality of bins containing the hot melt material.

6. The method according to any one of the preceding claims, comprising a step (d), prior to step (b), of retrieving the hot melt material from a storage device comprising a plurality of bins, at least one of which contains the hot melt material.

7. The method according to claim 6, wherein the plurality of bins have substantially the same configuration but the hot melt material within individual bins of the plurality of bins have a different configuration from one another.

8. The method according to claim 6 or 7, comprising a step (e), prior to step (d), of transporting the storage device from a first location to a second location that is closer to the manufacturing converting line.

9. The method according to any one of claims 1-5, comprising steps of:
(g) signaling, via a fill-level sensing device associated with a second hot melt material tank, a driverless vehicle to deliver a second hot melt material;
(h) delivering, via the driverless vehicle, the second hot melt material to the second hot melt material tank; and
(i) loading, via an end effector associated with the driverless vehicle, the second hot melt material into the second hot melt material tank.

10. The method according to claim 9, wherein the first hot melt material tank supplies a first manufacturing converting line, wherein the second hot melt material tank supplies a second manufacturing converting line, and wherein the first hot melt material is the same as the second hot melt material.

11. The method according to claim 9, wherein both of the first hot melt material tank and the second hot melt material tank supply a single manufacturing converting line, and wherein the first hot melt material is different from the second hot melt material.

12. The method according to claim 9 or 11, wherein the first hot melt material is different from the second hot melt material in composition and solid-state product form.

13. The method according to any one of claims 9, 11 and 12, wherein the first hot melt material comprises a first composition and a first mass, wherein the second hot melt material comprises a second composition and a second mass, and wherein a ratio of the first mass to the second mass is at least 5:1.

14. The method according to any one of claims 9 and 11-13, wherein the first hot melt material comprises a first composition and a first volume, wherein the second hot melt material comprises a second composition and a second volume, and wherein a ratio of the first volume to the second volume is at least 5:1.

15. The method according any one of claims 9-14, wherein each of the first hot melt material and the second hot melt material are stored in a bin associated with a storage device comprising a plurality of bins, wherein the bin containing the first hot melt material has a first unique identifier and wherein the bin containing the second hot melt material has a second unique identifier so that the driverless vehicle can accurately complete steps (b), (c), (e), and (f).
